# EUROPEAN PATENT APPLICATION

(11) **EP 1 988 172 A1**
(43) Date of publication of application: **05.11.2008**
(21) Application number: 07714532.4
(22) Date of filing: 20.02.2007
(51) Int. Cl.: C12P 13/02

(54) **PROCESS FOR PRODUCING (METH)ACRYLAMIDE**

(30) Priority: 24.02.2006 JP 2006048549; 28.02.2006 JP 2006052371
(71) Applicant: Mitsui Chemicals, Inc., Tokyo 105-7117 (JP)
(72) Inventor: ABE, Takeya, Takaishi-shi, Osaka 592-8501 (JP); FUKUDA, Takeshi, Takaishi-shi, Osaka 592-8501 (JP)
(74) Representative: Wytenburg, Wilhelmus Johannes
(86) International application number: PCT/JP2007/053006
(87) International publication number: WO 2007/097292

(57) **Abstract**

A process for producing high-quality (meth)acrylonitrile efficiently by an action of a microbial catalyst or the like that contains nitrile hydratase is provided.

The present invention is directed to a process for producing (meth) acrylamide from (meth) acrylonitrile in an aqueous solution that is put in a reactor and relates to one process for producing (meth) acrylamide that includes the steps of (A) to (C) : (A) adding to the reactor a microorganism or a processed product of the microorganism that contains nitrile hydratase; (B) adding to the reactor an alkali; and (C) adding to the reactor (meth) acrylonitrile, in which an addition line used in the step (A), an addition line used in the step (B), and an addition line used in the step (C) are different from one another, and to another process for producing (meth)acrylamide from (meth)acrylonitrile by an action of the microorganism or processed product of the microorganism that produces nitrile hydratase, in which a mixture of (meth)acrylonitrile and water is added to an aqueous solution that is put in a reactor and contains an alkali and a microorganism or a processed product of the microorganism that produces nitrile hydratase through an addition line of the reactor.

## Description

### Technical Field

The present invention relates to a process for producing (meth) acrylamide, more specifically, to a process for producing (meth)acrylamide with excellent properties through hydrating efficiently (meth) acrylonitrile by an action of a microorganism or the like that contains nitrile hydratase.

### Background Art

As a prevalent process for producing (meth)acrylamide, there may be mentioned a process for hydrating (meth)acrylonitrile. Examples of known processes include a hydration process using a metallic copper catalyst such as Raney copper and a hydration process using as a catalyst a microorganism, a processed product of the microorganism or the like that contains nitrile hydratase.

Among these processes, the process for producing (meth) acrylamide using as a catalyst a microorganism or the like that contains nitrile hydratase has received attention as an industrial process (for example, see Patent Document 1), because the process provides a high (meth)acrylonitrile conversion and a high selectivity as compared with a conventional process where hydration is carried out using a metallic copper catalyst or the like.

In order to produce high-quality (meth)acrylamide efficiently using as a catalyst a microorganism or the like that contains nitrile hydratase, hydration efficiency is required to be enhanced as much as possible. In addition, the source (meth)acrylonitrile is required to be used efficiently as much as possible.
Patent Document 1: Japanese Patent Laid-Open Publication No. 2001-340091.

### [Disclosure of the Invention]

### [Problems to Be Solved by the Invention]

An object of the present invention is to provide a process for producing high-quality (meth)acrylamide efficiently by an action of a microbial catalyst or the like that contains nitrile hydratase.

### [Means for Solving the Problems]

The present inventors have studied the above problem and have found that high-quality (meth)acrylamide can be produced by adding (meth)acrylonitrile and others that are used in the production in a specific manner when (meth) acrylamide is produced in an alkali-containing aqueous solution put in a reactor by allowing (meth)acrylonitrile to be hydrated by an action of a microorganism or a processed product of the microorganism that contains nitrile hydratase. The present invention has been accomplished based on this finding.

In other words, a first process for producing (meth)acrylamide of the present invention is a process for producing (meth)acrylamide from (meth)acrylonitrile in an aqueous solution put in a reactor, and includes the following steps of (A) to (C):
(A) adding to the reactor a microorganism or a processed product of the microorganism that contains nitrile hydratase;
(B) adding to the reactor an alkali; and
(C) adding to the reactor (meth)acrylonitrile, in which
an addition line used in the step (A), an addition line used in the step (B), and an addition line used in the step (C) are different from one another.

Furthermore, a second process for producing (meth)acrylamide of the present invention is characterized in that: to an aqueous solution that is put in a reactor and contains an alkali and a microorganism or a processed product of the microorganism that produces nitrile hydratase, a mixture of (meth) acrylonitrile and water is added through an addition line of the reactor so as to produce (meth)acrylamide from (meth) acrylonitrile by an action of the microorganism or processed product of the microorganism that contains nitrile hydratase.

### [Effect of the Invention]

According to the present invention, high-quality (meth)acrylamide can be produced efficiently from (meth) acrylonitrile by an action of a microbial catalyst or the like that contains nitrile hydratase.

### [Best Mode for Carrying out the Invention]

The present invention is hereinafter described in detail.

Firstly, there will be explained a source material and the like used in the process for producing (meth) acrylamide according to the present invention.

### (Meth)acrylonitrile

There is not any limitation on (meth)acrylonitrile used in the present invention, but (meth)acrylonitrile freed from impurities is preferable.

As a method of removing impurities from (meth) acrylonitrile, there may be mentioned, for example, distillation purification, cleaning with an alkali aqueous solution, removal with ion exchange resins such as cation exchange resins and anion exchange resins, removal with active carbon, and the like.

The (meth)acrylonitrile processed as above provides (meth)acrylamide with still higher quality more efficiently.

In the present invention, when (meth) acrylonitrile is added in the form of an aqueous solution to a reactor, there is not any limitation on the concentration thereof. However, when an excessively large amount of (meth)acrylonitrile is supplied to the reactor, a large amount of catalyst to fulfill the reaction, a reactor excessively large in volume, and a heat exchanger excessively large in size to remove reaction heat are required, so that great expense is expected in the instrumentation. Hence the supply concentration of (meth)acrylonitrile is preferably selected as: in the case of acrylonitrile, acrylonitrile is added in such a manner that the theoretical concentration of product acrylamide with respect to the total weight of the reactant aqueous solution put in the reactor is in the range of from 40 to 80 wt% assuming that all of the acrylonitrile added is transformed into acrylamide, more specifically, acrylonitrile is preferably added in an amount of from 0.4 to 1.5 parts by weight with respect to 1 part by weight of water.

In the case of methacrylonitrile, methacrylonitrile is preferably added in such a manner that the theoretical concentration of product methacrylamide with respect to the total weight of the reactant aqueous solution put in the reactor is in the range of from 10 to 40 wt% assuming that all of the methacrylonitrile added is transformed into methacrylamide, more specifically, methacrylonitrile is preferably added in an amount of from 0.08 to 0.5 part by weight with respect to 1 part by weight of water contained in the above mixture.

Microorganism or the like that contains nitrile hydratase In the present invention, in an aqueous solution, the aforementioned (meth)acrylonitrile is used as a source material and is hydrated by using as a catalyst a microorganism or a processed product of the microorganism (hereinafter, referred to as "microorganism or the like" in some cases) that contains nitrile hydratase, so that (meth)acrylamide of the present invention can be obtained.

In the present invention, nitrile hydratase refers to an enzyme that has a capability of hydrolyzing a nitrile compound and producing a corresponding amide compound. There is not any particular limitation on the microorganism that contains nitrile hydratase as far as the microorganism produces nitrile hydratase that has a capability of hydrolyzing a nitrile compound and producing a corresponding amide compound and keeps the activity of nitrile hydratase in an acrylamide aqueous solution.

Specifically, as a preferred example, there may be mentioned a microorganism belonging to the genera Nocardia, Corynebacterium, Bacillus, thermophilic Bacillus, Pseudomonas, Micrococcus, Rhodococcus such as rhodochrous species, Acinetobacter, Xanthobacter, Streptomyces, Rhizobium, Klebsiella, Enterobacter, Erwinia, Aeromonas, Citrobacter, Achromobacter, Agrobacterium, and Pseudonocardia such as thermophila species.

A transformant obtained by expressing a nitrile hydratase gene cloned from the microorganism in an arbitrary host is also included in the microorganism of the present invention. While Escherichia coli is mentioned as a representative example of the arbitrary host referred herein, it is not particularly limited to Escherichia coli, but Bacillus such as Bacillus subtilis, and other microorganism strains such as yeast and actinomycete are also included. Examples thereof include MT-10822 (the strain has been deposited in National Institute of Bioscience and Human Technology, Ministry of International Trade and Industry, 1-3,
Higashi 1-chome, Tsukubashi, Ibaraki-ken (presently, International Patent Organism Depositary, National Institute of Advanced Industrial Science and Technology, Tsukuba Central 6, Higashi 1-1-1, Tsukubashi, Ibaraki-ken), on February 7, 1996 under Receipt No. FERM BP-5785 based on the Budapest Treaty and Regulations on the International Recognition of the Deposit of Microorganisms for the Purposes of Patent Procedure). The microorganism described herein may also include a transformant obtained by expressing a mutant nitrile hydratase that is further improved in acrylamide resistance, acrylonitrile resistance, and temperature resistance by displacing, deleting, canceling or inserting at least one of constitutional amino acids of the enzyme with other amino acids by using recombinant DNA technology.

Upon producing an amide compound by using the foregoing microorganisms, the microorganism or a processed product of the microorganismisgenerallyused. The microorganism may be prepared by utilizing a known ordinary method in the fields of molecular biology, bioengineering, and genetic engineering. For example, such a method may be mentioned that after the microorganism is planted in an ordinary liquid culture medium, such as an LB medium, an M9 medium and the like, the microorganism is grown at an appropriate culture temperature (generally from 20°C to 50°C, and possibly 50°C or higher for thermophile), and then the microorganism is separated and recovered from the culture liquid by centrifugal separation.

The processed product of the microorganism used in the present invention denotes an extract and a trituration product of the microorganism, a post-separated product obtained by separating and purifying a nitrile hydratase active fraction of the extract and trituration product, a fixed product obtained by fixing the microorganism, the extract, trituration product, or post-separated product of the microorganism on an appropriate carrier, and the like. These are included in the processed product of the microorganism used in the present invention as far as they have an activity of nitrile hydratase. These may be used in one kind or two or more kinds different from each other at the same time or alternately.

There is not any particular limitation on the method of feeding these microorganisms or the like into a reactor. These microorganisms or the like may be dissolved or suspended in water and added to the reactor. Alternatively, the microorganisms or the like may be preliminarily fed prior to reaction. These can be used in combination freely in accordance with the mode of reaction such as a suspended-bed mode, a fixed bed-mode, or a fluidized-bed mode.

Although the used amount of the foregoing microorganisms or the like depends on reaction conditions, kinds of catalysts, and forms thereof, it is generally from 10 to 50,000 ppm by weight and preferably from 50 to 30,000 ppm by weight in terms of the dry weight of the microorganisms or the like with respect to the total weight of the reaction solution in a reactor.

### Alkali

In the present invention, an alkali is used for the production of acrylamide. There is not any particular limitation on the alkali as far as the alkali exhibits basic when dissolved in water. Examples of the alkali include an alkali metal hydroxide such as sodium hydroxide or potassium hydroxide; an alkaline earth metal hydroxide such as calcium hydroxide; an alkali metal carbonate such as sodium carbonate; ammonia; an amine such as methylamine; and the like.

Among these alkalis, sodium hydroxide is preferable because it has a high water solubility and is industrially easily available.

The alkali may be put in a reactor, for example, as it is or as an alkali aqueous solution prepared by dissolving an alkali and conditioning the concentration adequately.

When the alkali is added as an aqueous solution, an aqueous solution generally from 0.01 to 20 wt% may be added.

### Other additives

Furthermore, in the present invention, for the purpose of controlling pH, may be added a strong acid such as sulfuric acid, nitric acid and hydrochloric acid, a weak acid such as carboxylic acid and phosphoric acid, a buffering agent such as phosphate, an inorganic salt such as nitrate and carbonate, an amide compound, and the like.

There is not any particular limitation on the method of adding these substances to the reactor and, for example, these substances may be added to the reactor as they are or after they are dissolved or suspended in water.

### Addition step (1)

In a first embodiment of the present invention, the aforementioned (meth)acrylamide, a microorganism or the like, alkali, water, and others are added to a reactor so as to hydrate (meth)acrylonitrile by an action of the foregoing microorganism or the like in an aqueous solution. The present invention is characterized by the method of adding these substances to the reactor.

A first process for producing (meth)acrylamide according to the present invention is characterized by including the steps of (A) to (C):
(A) adding to a reactor a microorganism or the like that contains nitrile hydratase;
(B) adding to the reactor an alkali; and
(C) adding to the reactor (meth)acrylonitrile, wherein
as to addition lines used in the step (A) of adding the microorganism or the like, in the step (B) of adding the alkali, and in the step (C) of adding (meth)acrylonitrile, each addition line is different from one another.

In the above description, "each addition line is different from one another" denotes that not only each addition inlet of the above substances, but also a pipe or the like that connects a tank or the like that stores the substances to be added and the addition inlet is different from one another.

A common addition inlet has generally been used conventionally, and parts of the pipes or the like of the addition lines have been used in common in some cases so as to add the aforementioned substances such as (meth)acrylonitrile to the reactor and to hydrate (meth)acrylonitrile. Hence, in the step of adding the microorganism or the like, when the microorganism or the like and alkali are simultaneously added, the microorganism or the like comes into direct contact with the alkali or a highly concentrated alkali aqueous solution in the addition line before the microorganism or the like is added to the reactor. Furthermore, even in the case where they are not simultaneously added, the microorganism or the like sometimes comes into contact with the alkali or alkali aqueous solution remained in the addition line. This causes a problem that the catalytic action that the microorganism or the like originally possesses is damaged because of the alkali.

However, when the addition line used in the step (A) of adding microorganism or the like, and the addition line used in the step (B) of adding an alkali are made different from each other in accordance with the first embodiment of the present invention, the problem of damage of the catalytic action caused by the added alkali does not occur, thereby allowing the catalytic action that microorganism or the like originally possesses to be expressed to the greatest extent in the reaction system in the reactor.

In the case where (meth)acrylonitrile and microorganism or the like are simultaneously added, (meth)acrylonitrile sometimes comes into direct contact with the microorganism or the like before they are added to a reaction aqueous solution in a reactor. Furthermore, even in the case where they are not simultaneously added, the microorganism or the like sometimes comes into contact with (meth)acrylonitrile remained. Because of this, the catalytic activity of the microorganism or the like is lowered by coming into contact with (meth) acrylonitrile itself, thereby sometimes causing the reaction not to proceed.

However, when the addition line used in the step (A) of adding microorganism or the like and the addition line used in the step (C) of adding (meth)acrylonitrile are made different from each other in accordance with the aforementioned first embodiment, the problem of damage of the catalytic action caused by the added (meth) acrylonitrile does not occur, thereby allowing the catalytic action that microorganism or the like originally possesses to be expressed to the greatest extent in the reaction system in the reactor.

Furthermore, in the case where a common addition line is used for the addition of (meth)acrylonitrile and the addition of an alkali, (meth)acrylonitrile itself sometimes comes into direct contact with the alkali added, side reactions such as (meth)acrylonitrile polymerization sometimes proceed in the addition line, which causes side reaction products to be sometimes mixed as impurities in the reaction aqueous solution put in the reactor or polymerization products to be formed in the addition line and clog the pipes in some cases.

However, when the addition line used in the step (B) of adding an alkali and the addition line used in the step (C) of adding (meth)acrylonitrile are made different from each other in accordance with the present invention, impurities are not mixed into the reaction aqueous solution put in the reactor and the pipes are not clogged with the polymerization products. Because of this, without wasting (meth)acryliamide that is used as a source material and also without damaging the catalytic activity of nitrile hydratase that the catalyst originally possesses, high-purity (meth)acrylamide can be produced efficiently.

In addition, in the case where the addition of (meth) acrylonitrile in the step (C) is carried out using a mixture of (meth)acrylonitrile and water, impurities derived from (meth)acrylonitrile polymerization are not mixed in the reactor and the addition lines are more unlikely to be clogged, thereby producing high-purity (meth)acryliamide still more efficiently.

Note that, a small amount of by-products may possibly be contained in the above mixture of (meth)acrylonitrile and water in some cases, but it is preferred that a microorganism or a processed product of the microorganism that produces nitrile hydratase should not be contained in the mixture. Such a mixture is likely to prevent the catalytic activity of the microorganism or the like from being lowered by a contact between the microorganism or the like and highly concentrated (meth)acrylonitrile.

### Addition step (2)

In a second embodiment of the present invention, the aforementioned (meth)acrylonitrile, microorganism or the like, alkali, water, and others are added to a reactor so as to hydrate (meth)acrylonitrile by an action of the microorganism or the like in an aqueous solution. The second embodiment is characterized by the method of adding (meth)acrylonitrile to the reactor.

A second process for producing (meth)acrylamide according to the present invention is characterized in that a mixture of (meth)acrylonitrile and water is added through an addition line that is equipped with a reactor when (meth) acrylonitrile is added to the reactor.

The addition line in the present invention includes an addition inlet of the reactor for the aforementioned substances and a pipe that connects the addition inlet and a storage tank of the mixture of (meth)acrylonitrile and water.

Conventionally, when the source (meth)acrylonitrile is added to the reactor from the storage tank, the (meth)acrylonitrile is generally added without diluted.

However, from the viewpoint of preventing impurities from being mixed into the reactor and avoiding the loss of the source (meth)acrylonitrile by suppressing (meth)acrylonitrile polymerization in the addition line and preventing the addition line from being clogged, it is preferably that the mixture of (meth) acrylonitrile and water is added through the addition line in accordance with the second embodiment described above upon adding (meth)acrylonitrile to the reactor.

In the mixture of (meth)acrylonitrile and water, there may be possibly contained by-products in an small amount, but the mixture preferably does not contain a microorganism or a processed product of the microorganism that produces nitrile hydratase. Such a mixture is likely to prevent the catalytic activity of the microorganism or the like from being lowered by a contact between the microorganism or the like and highly concentrated (meth)acrylonitrile.

### Reaction conditions

In the present invention, through the aforementioned addition step, (meth)acrylonitrile, a microorganism or the like, an alkali, water, and others are added to a reactor, so that they react in an aqueous solution in the reactor, thereby producing (meth)acrylamide.

In the above reaction, the reaction time is generally in the range of from 1 to 80 hours per one reactor and preferably from 2 to 40 hours, although it depends on conditions such as the amount of a catalyst used and temperature.

The hydration reaction is carried out generally under normal or around normal pressure, but may be carried out under pressure so as to increase the solubility of the nitrile compounds into the water medium. The reaction temperature is not particularly limited as long as it is above the freezing point of the water medium, but generally is in the range of preferably from 0 to 50°C and more preferably from 10 to 40°C. Furthermore, the reaction may be carried out while the reaction solution is in a slurry state where the reaction product is crystallized therein. The pH of the reaction solution on the hydration reaction is not particularly limited as long as nitrile hydratase is kept active, but is in the range of preferably from pH 6 to pH 10 and more preferably from pH 7 to pH 9.

### Purification method of (meth)acrylamide

As a method of purifying (meth)acrylamide out of the (meth)acrylamide aqueous solution obtained in the present invention, there may be mentioned a method of removing catalysts and impurities with active carbon or ion exchange resins. For example, as described in Japanese Patent Laid-Open Publication No. 2001-270857, (meth)acrylamide can be purified easily by contacting the aqueous solution with active carbon in an acid condition.

### Production of (meth)acrylamide polymers

By using the (meth)acrylamide produced in the present invention, (meth)acrylamide may be homopolymerized or copolymerized, or it may be copolymerized with other monomers.

Examples of the other monomers copolymerizable with (meth)acrylamide include:
an unsaturated carboxylic acid such as acrylic acid, methacrylic acid, itaconic acid, maleic acid and fumaric acid, and salts thereof;
vinylsulfonic acid, styrenesulfonic acid, acrylamide methylpropane sulfonic acid, and salts thereof;
an alkylaminoalkyl ester of (meth)acrylic acid such as N,N-dimethylaminoethyl methacrylate, N,N-diethylaminoethyl methacrylate, and N,N-dimethylaminoethylacrylate acrylic acid, and quaternary ammonium derivatives thereof;
an N,N-dialkylaminoalkyl (meth)acrylamide such as N, N-dimethylaminopropyl methacrylamide and N,N-dimethylaminopropyl acrylamide and quaternary ammonium derivatives thereof;
a hydrophilic acrylamide such as acetone acrylamide, N,N-dimethylacrylamide, N,N-dimethylmethacrylamide, N-ethylmethacrylamide, N-ethylacrylamide, N,N-diethylacrylamide, and N-propylacrylamide; N-acryloylpyrrolidine, N-acryloylpiperidine and
N-acryloylmorpholine; hydroxyethylmethacrylate, hydroxyethylacrylate, hydroxypropylmethacrylate and hydroxypropylacrylate; methoxypolyethyleneglycol (meth)acrylate and N-vinyl-2-pyrrolidone;
an N-alkyl(meth)acrylamide derivative such as N,N-di-n-propylacrylamide, N-n-butylacrylamide, N-n-hexylacrylamide, N-n-hexylmethacrylamide, N-n-octylacrylamide, N-n-octylmethacrylamide, N-tert-octylacrylamide, N-dodecylacrylamide, and N-n-dodecylmethacrylamide;
an N- (w-glycidoxyalkyl) (meth) acrylamide derivative such as N,N-diglycidylacrylamide, N,N-diglycidylmethacrylamide, N-(4-glycidoxybutyl) acrylamide, N-(4-glycidoxybutyl) methacrylamide, N-(5-glycidoxypentyl) acrylamide, and N-(6-glycidoxyhexyl) acrylamide;
a (meth)acrylate derivative such as methyl(meth)acrylate, ethyl(meth)acrylate, butyl(meth)acrylate, lauryl(meth)acrylate, 2-ethylhexyl(meth)acrylate, and glycidyl(meth)acrylate;
an olefin such as acrylonitrile, methacrylonitrile, vinylacetate, vinylchloride,vinylidenechloride,ethylene,propylene and butene, styrene, α-methylstyrene, butadiene, and isopurene; and the like.

These other monomers may be used in one kind or in combination of two or more kinds.

Furthermore, acrylamide and methacrylamide may be copolymerized with these other monomers.

There is not any particular limitation on the mixing ratio when (meth)acrylamide is copolymerized with these other monomers, but with respect to 100 mol of (meth) acrylamide, the other monomers are generally 100 mol or less and preferably 50 mol or less.

As a method of polymerizing these monomers, there may be mentioned, for example, aqueous solution polymerization, emulsion polymerization, and the like.

Among these, in the case of aqueous solution polymerization, the total concentration of (meth) acrylamide and the other monomers that are optionally added is generally selected from 5 to 90 wt%.

As a polymerization initiator, a radical polymerization initiator may be used.

Examples of the radical polymerization initiator include: a peroxide such as potassium persulfate, ammonium persulfate, hydrogen peroxide, and benzoyl peroxide; an azo free-radical initiator such as azobisisobutylonitrile, 2,2'-azobis(4-amidinopropane) dihydrochloride, and 4,4'-azobis(sodium 4-cyanovalerate); and a so-called redox catalyst that is a combination of the aforementioned peroxide and a reducing agent such as sodium bisulfite, triethanolamine, and ammonium ferrous sulfate.

The above polymerization initiator may be used in one kind or in combination of two or more kinds. The amount of the polymerization initiator is generally in the range of from 0.001 to 5 wt% with respect to the total weight of the monomers.

The polymerization temperature is in the range of generally from -10 to 120°C and preferably from 0 to 90°C. The polymerization temperature is not always required to be kept constant and may be changed as appropriate with the advancement of polymerization. Generally, polymerization heat is generated with the advancement of polymerization and the polymerization temperature is likely to elevate, so that if necessary cooling is carried out in some cases.

There is not any particular limitation on the polymerization atmosphere, but from the viewpoint of smooth advancement of polymerization, for example, polymerization in an atmosphere of an inert gas such as a nitrogen gas is preferable.

There is not any particular limitation on the polymerization time, but it is in the range of generally from 1 to 20 hours.

Furthermore, there is not any particular limitation on the pH of the aqueous solution upon polymerization, but if necessary the pH may be controlled upon polymerization. As a pH controlling agent that is usable on that occasion, there may be mentioned an alkali such as sodium hydroxide, potassium hydroxide, and ammoniumhydroxide; a mineral acid such as phosphoric acid, sulfuric acid, and hydrochloric acid; an organic acid such as formic acid and acetic acid; and the like.

There is not any particular limitation on the molecular weight of a polymer obtained in the present invention, but it is in the range of generally from 100,000 to 50,000,000 and preferably from 500,000 to 30,000,000.

Thus obtained (meth) acrylamide polymer exhibits an extremely enhanced water solubility, having a sufficiently high molecular weight, and being excellent in color, because the (meth) acrylamide obtained in the present invention is excellent in qualities. Therefore, the (meth)acrylamide polymer can be suitably used as a flocculant, a paper-making additive, a petroleum scavenger, or the like.

### Example

The present invention will be described below in more detail with reference to examples, but the present invention should not be construed as being limited by the examples.

### Example 1

### Charging separately microorganism, alkali, and acrylonitrile Culture of microorganism containing nitrile hydratase

In accordance with the method described in Example 1 of Japanese Patent Laid-Open Publication No. 2001-340091, No. 3 clone microorganism was prepared. Similarly, a wet microorganism containing nitrile hydratase was obtained by culture in accordance with the method described in the same Example 1.

### Production of acrylamide

A 1 liter glass flask equipped with a stirrer was prepared as a first reactor, and a Teflon (trade name) tube 20 m long with an inside diameter of 5 mm was prepared as a second reactor. In the first reactor were preliminarily charged 400 g of water.

The wet microorganism obtained by the above culture method was suspended in pure water in an amount of 12%. The resulting suspension was continuously fed at a rate of 11 g/hr while the inside the first reactor was stirred. Acrylonitrile was fed at a rate of 31 g/hr and pure water was fed at a rate of 38 g/hr continuously. Furthermore, in order to adjust the reaction pH at 7.5 to 8.5, a 0.1 M NaOH aqueous solution was continuously fed. Each of these source materials was fed from each storage tank through a line separated from each other, so that each source material did not come into contact with the other source materials until it was fed into the reactor. In order to keep constant the liquid level in the first reactor, the reaction solution was continuously drained out of the first reactor at a rate of 80 g/hr and was continuously fed into the second reactor, so that the reaction was continued in the second reactor.

Both first and second reactors were immersed in a 10 to 20°C water bath so as to regulate the liquid temperature inside each reactor at 15°C.

On the second day after the operation started, the reaction solution of each reactor was sampled so as to analyze with HPLC; the percent of conversion to acrylamide at the outlet of the first reactor was 93%, and the acrylonitrile concentration at the outlet of the second reactor was below the detection limit (100 ppm by weight or less). Even after another 4-day continuous operation, the percent of conversion of each reactor was hardly changed. There occurred no trouble on the feed lines of the source materials and the operation was performed stably.

### Example 2

In Example 2, after a mixture of acrylonitrile and water was added to a reactor, acrylonitrile was hydrated by an action of nitrile hydratase. A microorganism, an alkali, and the mixture of acrylonitrile and water were charged separately.

The microorganism containing nitrile hydratase was cultured similarly to that of Example 1.

### Production of acrylamide

A 2 liter glass flask equipped with a stirrer was prepared as a first reactor, and a stainless steel (SUS304) tube 30 m long with an inside diameter of 6 mm was prepared as a second reactor. In the first reactor were preliminarily charged 800 g of water.

The wet microorganism obtained by the above culture method was suspended in pure water in an amount of 12%. The resulting suspension was continuously fed at a rate of 22 g/hr while the inside the first reactor was stirred. Acrylonitrile was fed at a rate of 62 g/hr and pure water was fed at a rate of 76 g/hr continuously. Acrylonitrile and pure water were preliminarily mixed and the resulting mixture was added to the reactor. Furthermore, in order to adjust the reaction pH at 7.5 to 8.5, a 0.1 M NaOH aqueous solution was continuously fed. Among these source materials, acrylonitrile and pure water were fed through the same line, and the others were fed from each storage tank through a line separated from each other. In order to keep constant the liquid level in the first reactor, the reaction solution was continuously drained out of the first reactor at a rate of 160 g/hr and was continuously fed into the second reactor, so that the reaction was continued in the second reactor.

Both first and second reactors were immersed in a 10 to 20°C water bath so as to regulate the liquid temperature inside each reactor at 15°C.
On the second day after the operation started, the reaction solution of each reactor was sampled so as to analyze with HPLC; the percent of conversion to acrylamide at the outlet of the first reactor was 92%, and the acrylonitrile concentration at the outlet of the second reactor was below the detection limit (100 ppm by weight or less) . Even after another 20-day continuous operation, the percent of conversion of each reactor was hardly changed. There occurred no trouble on the feed lines of the source materials and the operation was performed stably.

### Comparative Example 1

### Charging microorganism and alkali through the same line

The same operation as in Example 1 was performed except that a 2.5 wet% catalyst suspension and a 0.1 M NaOH aqueous solution were fed into a reactor through the same line. As a result, the acrylonitrile conversion was 8% in the first reactor on the second day. The reaction hardly proceeded. The reaction solution was separated into two layers of an acrylonitrile phase and a water phase.

### Comparative Example 2

### Charging microorganism and acrylonitrile through the same line

The same operation as in Example 1 was performed except that a 2.5 wt% catalyst suspension and acrylonitrile were fed into a reactor through the same line. As a result, the acrylonitrile conversion was 11% in the first reactor on the second day. The reaction hardly proceeded. The reaction solution was separated into two layers of an acrylonitrile phase and a water phase.

### Comparative Example 3

### Charging acrylonitrile and alkali through the same line

The same operation as in Example 1 was performed except that acrylonitrile and a 0.1 M NaOH aqueous solution were fed into a reactor through the same line. As a result, the acrylonitrile conversion was 88% in the first reactor on the second day, but depositions were found on the feed line. On the fourth day, the line was found to be clogged.

### Reference Example 1

In Reference Example 1, after acrylonitrile was added as it was to a reactor, acrylonitrile was hydrated by an action of nitrile hydratase. The same operation as in Example 2 was performed except that each of acrylonitrile and pure water was fed through a line separated from each other. As a result, the acrylonitrile conversion was 93% in the first reactor on the second day similarly to that of Example 2, but depositions were found on the feed line of acrylonitrile on the seventh day. On the tenth day, the line was found to be clogged.

## Claims

1. A process for producing (meth)acrylamide from (meth)acrylonitrile in an aqueous solution put in a reactor comprising the following steps of (A) to (C):
(A) adding to the reactor a microorganism or a processed product of the microorganism that contains nitrile hydratase;
(B) adding to the reactor an alkali; and
(C) adding to the reactor (meth)acrylonitrile, wherein
an addition line used in the step (A), an addition line used in the step (B), and an addition line used in the step (C) being different from one another.

2. The process for producing (meth)acrylamide according to claim 1, wherein
addition of (meth) acrylonitrile in the step (C) is performed by using a mixture of (meth)acrylonitrile and water.

3. The process for producing (meth)acrylamide according to claim 2, wherein
the mixture of (meth)acrylonitrile and water contains no microorganism or processed product of the microorganism that produces nitrile hydratase.

4. A process for producing (meth)acrylamide comprising:
adding into an aqueous solution that is put in a reactor and contains an alkali and a microorganism or a processed product of the microorganism that produces nitrile hydratase, a mixture of (meth)acrylonitrile and water through an addition line of the reactor so as to produce (meth)acrylamide from (meth)acrylonitrile by an action of the microorganism or processed product of the microorganism that produces nitrile hydratase.

5. The process for producing (meth)acrylamide according to claim 4, wherein
the mixture of (meth)acrylonitrile and water contains no microorganism or processed product of the microorganism that produces nitrile hydratase.
